# DEMANDE DE BREVET EUROPEEN

(11) **EP 4 732 765 A1**
(43) Date de publication de la demande: **29.04.2026**
(21) Numéro de dépôt: 25211549.8
(22) Date de dépôt: 28.10.2025
(51) Int. Cl.: A61B 5/024, A61B 5/16, A61B 5/00, A61B 5/18, A61B 5/01, A61B 5/0533, A61B 5/12, A61B 5/145, A61B 5/369, A61B 5/389

(54) **PROCÉDÉ DE DÉTERMINATION D'ÉTATS PHYSIOLOGIQUES D'UN OPÉRATEUR, ET PROGRAMME D'ORDINATEUR ET SYSTÈME DE DÉTERMINATION ASSOCIÉS**

(30) Priorité: 28.10.2024 FR 2411755
(71) Demandeur: THALES, 92190 Meudon (FR)
(72) Inventeur: NIAUSSAT, Victor, 33700 Mérignac (FR); BERTHELOT, Bastien, 33700 Mérignac (FR)
(74) Mandataire: Lavoix

(57) **Abrégé**

La présente invention concerne un procédé de détermination d'états physiologiques d'un operateur une première étape préliminaire (110) de détermination d'une matrice de correspondance entre un modèle mathématique permettant de déterminer un état physiologique de l'opérateur et un ensemble de types de données nécessaires à la mise en œuvre du modèle mathématique.

Le procédé comprenant en outre les étapes suivantes :
- vérification (220) de la disponibilité de chaque capteur (12) ;
- lorsqu'un capteur (12) est indisponible, exclusion (240) de considération de chaque modèle mathématique utilisant le type de données correspondant à ce capteur (12), conformément à la matrice de correspondance ;
- détermination (260) des états physiologiques de l'opérateur à partir des modèles mathématiques non-exclus de considération.

## Description

La présente invention concerne un procédé de détermination d'états physiologiques d'un opérateur.

La présente invention concerne également un programme d'ordinateur et un système de détermination associés à ce procédé de détermination.

Le domaine technique de l'invention est celui de la surveillance des opérateurs évoluant dans un contexte opérationnel critique.

L'invention peut être ainsi utilisée dans tout domaine à haute sensibilité dans lequel la surveillance des états physiologiques d'un opérateur présente un intérêt. Cela est le cas notamment dans le domaine aéronautique, aérospatial, ferroviaire, nucléaire, de la médecine, etc.

De manière connue en soi, la surveillance des états physiologiques d'un opérateur est un moyen d'analyser et d'évaluer son comportement cognitif en conditions opérationnelles, voire en dehors de ces conditions, notamment pour un suivi longitudinal.

Généralement, une telle analyse s'appuie sur des moyens permettant la capture et l'analyse des paramètres physiologiques et cognitifs, complétés par des paramètres de contexte.

Les données physiologiques peuvent présenter tout paramètre relatif à la vitalité du corps humain tel que la température corporelle, la fréquence cardiaque, la saturation en oxygène, ou encore la fréquence respiratoire.

Ces données sont généralement issues d'une pluralité de capteurs spécifiques optimisés pour mesurer celles-ci. En outre, ces données sont généralement associées à des traitements du signal de type filtrage permettant de réduire, voire d'éliminer les différents bruits des signaux susceptibles de dégrader les marqueurs à observer. Il s'agit d'apporter le bon niveau de traitement du signal qui va permettre d'obtenir un signal suffisamment propre pour être ensuite utilisé dans les modèles et obtenir une performance de détection ou de prédiction au niveau souhaité.

Selon les méthodes de l'état de la technique, le problème de surveillance des données physiologiques d'un opérateur est résolu en utilisant des moyens traditionnels tels que des capteurs spécifiques, des modèles de détection ou de prédiction et des algorithmes de traitement des signaux issus des capteurs.

Ces solutions reposent principalement sur l'extraction de caractéristiques intéressantes à partir des signaux physiologiques, suivie de l'utilisation d'algorithmes de détection ou de prédiction, tels que des algorithmes d'intelligence artificielle, pour séparer au mieux la détection ou la prédiction des différents états physiologiques.

Selon l'état de la technique, dans un premier temps, les capteurs spécifiques sont utilisés pour capturer les signaux physiologiques tels que la fréquence cardiaque, la saturation en oxygène, la température corporelle, etc. Ensuite, des techniques de traitement du signal sont appliquées sur ces signaux pour réduire les bruits et les perturbations indésirables afin d'obtenir des signaux de meilleure qualité.

Lorsque ces signaux sont prétraités, l'état de la technique propose d'extraire des caractéristiques pertinentes à partir de ces signaux physiologiques. Ces caractéristiques peuvent inclure des paramètres tels que la variabilité de la fréquence cardiaque, les pics de fréquence cardiaque, la variation de la saturation en oxygène, etc. L'objectif ici est de sélectionner les caractéristiques les plus discriminantes qui permettent de différencier efficacement les différents états physiologiques.

Ensuite, ces caractéristiques sont utilisées comme entrée des algorithmes de détection ou de prédiction, tels que des algorithmes d'intelligence artificielle, qui sont entrainés à reconnaitre les schémas et les motifs caractéristiques de chaque état physiologique. Ces algorithmes peuvent inclure des techniques de classification et de régression, ou même des réseaux neuronaux pour effectuer la détection ou la prédiction des états physiologiques spécifiques.

Les solutions de l'état de la technique présentent toutefois un certain nombre de défauts.

Tout d'abord, les solutions traditionnelles sont souvent limitées par la disponibilité et la fiabilité des capteurs.

En particulier, en cas de défaillance d'un capteur, l'analyse des signaux physiologiques peut être compromise, ce qui peut entrainer des erreurs de détection ou de prédiction ou une incapacité à détecter des états physiologiques anormaux.

De plus, les solutions existantes ne prennent pas en compte de manière optimale la gestion de la disponibilité des capteurs. En cas de perte d'un capteur, il est souvent nécessaire d'arrêter ou de recalibrer complètement le système, ce qui entraine des interruptions dans la surveillance et des délais pour rétablir le fonctionnement normal.

Les solutions existantes manquent donc de flexibilité et de capacité à gérer les cas où les modèles de détection ou de prédiction des états physiologiques sont indisponibles en raison des capteurs défaillants.

Cela signifie qu'en l'absence de certains capteurs, il est difficile, voire impossible, d'obtenir des résultats fiables sur les états physiologiques de l'opérateur.

La présente invention a pour but de remédier à ces inconvénients et de proposer une solution permettant de déterminer des états physiologiques d'un opérateur de manière fiable même lorsqu'un ou plusieurs capteurs ne sont plus disponibles.

En outre, la solution est particulièrement flexible dans la mesure où elle peut s'adapter facilement à une éventuelle perte d'un ou de plusieurs capteurs.

À cet effet, l'invention concerne un procédé de détermination d'états physiologiques d'un operateur ;
le procédé comprenant une première étape préliminaire de détermination d'une matrice de correspondance entre un modèle mathématique permettant de déterminer un état physiologique de l'opérateur et un ensemble de types de données nécessaires à la mise en œuvre du modèle mathématique ;
le procédé comprenant en outre les étapes suivantes :
   - vérification de la disponibilité de chaque capteur permettant de fournir un type de données ;
   - lorsqu'un capteur est indisponible, exclusion de considération de chaque modèle mathématique utilisant le type de données correspondant à ce capteur, conformément à la matrice de correspondance ;
   - détermination des états physiologiques de l'opérateur à partir des modèles mathématiques non-exclus de considération.

Les états physiologiques sont avantageusement tous différents.

Suivant d'autres caractéristiques avantageuses de l'invention, le procédé comprend une ou plusieurs des caractéristiques suivantes, prise(s) isolément ou suivant toutes les combinaisons techniquement possibles :
- le procédé comprenant en outre une étape de substitution d'au moins un modèle mathématique exclu de considération par un sous-modèle mathématique permettant de déterminer le même état physiologique à partir d'un ensemble de types de données sans le type de données du capteur indisponible correspondant ;
- le procédé comprenant en outre une deuxième étape préliminaire de détermination d'une matrice d'incidence indiquant le niveau d'incidence de chaque état physiologique sur chaque autre état physiologique ;
- chaque niveau d'incidence correspond à la probabilité de passage d'un état physiologique à un autre état physiologique ;
- le procédé comprenant en outre une étape de détermination d'au moins un état physiologique correspondant à un modèle mathématique exclu de considération, par un modèle d'agrégation utilisant le ou chaque état physiologique induisant cet état physiologique correspondant au modèle mathématique exclu de considération, conformément à la matrice d'incidence ;
- ledit état physiologique correspondant au modèle mathématique exclu de considération est déterminé avec un niveau de confiance déterminé en fonction du niveau d'incidence du ou de chaque état physiologique utilisé par le modèle d'agrégation ;
- chaque état physiologique est choisi dans le groupe comprenant au moins :
   - l'hypoxie ;
   - la perte de conscience ;
   - le stress ;
   - la fatigue ;
   - la désorientation spatiale ;
   - le voile noir ;
   - la déshydratation ;
   - la charge mentale ;
   - la divagation attentionnelle ;
   - l'hyperventilation ;
   - l'hypoglycémie ;
   - la tunnelisation visuelle ou attentionnelle ;
- chaque type de données est choisi dans le groupe comprenant au moins :
   - Rythme cardiaque, notamment à des fréquences différentes ;
   - Saturation en oxygène ;
   - Électroencéphalogramme ;
   - Fréquence respiratoire ;
   - Température corporelle ;
   - Images ;
   - Données acoustiques ;
   - Activité oculaire ;
   - Position et accélération de la tête ;
   - Niveaux de glucose sanguin ;
   - Conductance cutanée ;
   - Activité musculaire ;
   - Photopléthysmographie ;
   - Spectroscopie proche infra-rouge fonctionnelle.

L'invention concerne également un programme d'ordinateur comportant des instructions logicielles qui, lorsqu'elles sont exécutées par un ordinateur, mettent en œuvre un procédé tel que défini ci-dessus.

L'invention concerne enfin un système de détermination d'états physiologiques d'un opérateur comprenant des moyens techniques configurés pour mettre en œuvre un procédé tel que défini ci-dessus.

L'invention apparaitra plus clairement à la lecture de la description qui va suivre, donnée uniquement à titre d'exemple non limitatif, et faite en référence aux dessins annexés dans lesquels :
- [Fig.1] la figure 1 est une vue schématique d'un système de détermination selon l'invention ;
- [Fig.2] la figure 2 est un organigramme d'un procédé de détermination d'états physiologiques d'un opérateur, le procédé de détermination étant mis en œuvre par le système de détermination de la figure 1 ;
- [Fig.3] [Fig.4] [Fig.5] [Fig.6] [Fig.7] [Fig.8] [Fig.9] Les figures 3 à 9 sont différentes illustrations de la mise en œuvre de différentes étapes du procédé de la figure 2.

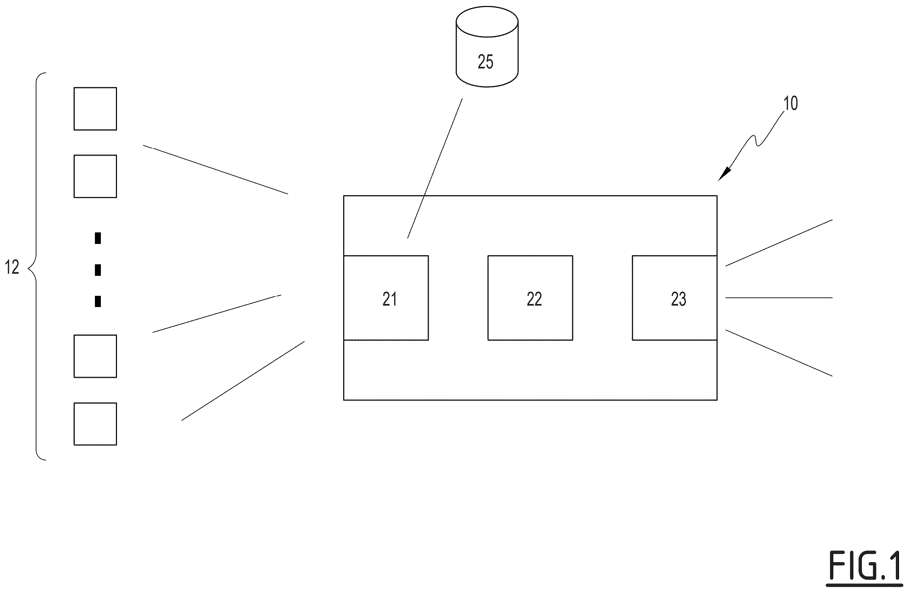

On a en effet représenté sur la figure 1 un système de détermination d'états physiologiques d'un opérateur selon l'invention.

Par opérateur, on entend toute personne opérant une machine, un véhicule ou tout autre système sensible. Ainsi, par exemple, un tel opérateur pilote un aéronef, tel qu'un avion. Dans un tel cas, cet opérateur est un pilote d'avion.

Alternativement, l'opérateur présente tout autre membre d'équipage ou alors un opérateur au sol faisant la maintenance d'un aéronef.

Par aéronef, on entend tout engin volant pilotable à partir du cockpit de celui-ci, comme cela est le cas par exemple d'un avion ou d'un hélicoptère, ou alors à distance de celui-ci, comme cela est le cas par exemple d'un drone.

De manière générale, la notion de l'opérateur peut s'appliquer à toute autre personne effectuant une mission critique, par exemple dans le domaine de transport (ferroviaire ou poids lourd ou tout transport par exemple) ou dans le domaine nucléaire ou spatial, ou dans la médecine.

Le système de détermination 10 selon l'invention permet de déterminer une pluralité d'états physiologiques différents de cet opérateur.

En particulier, par état physiologique, on entend tout état de l'opérateur susceptible d'influencer son travail physique ou son travail cognitif.

Chaque état physiologique est par exemple choisi dans le groupe comprenant au moins :
- l'hypoxie ;
- la perte de conscience ;
- le stress ;
- la fatigue ;
- la désorientation spatiale ;
- le voile noire (ou G-LOC en anglais) ;
- la déshydratation ;
- la charge mentale ;
- la divagation attentionnelle ;
- l'hyperventilation ;
- l'hypoglycémie ;
- la tunnelisation visuelle ou attentionnelle.

Le système de détermination 10 permet de déterminer un état physiologique de l'opérateur à partir d'une pluralité de données fournies par une pluralité de capteurs 12.

Ces capteurs 12 sont par exemple disposés sur le corps ou alors près du corps de l'opérateur ou alors dans l'endroit de l'exercice de ses activités tel que le cockpit d'aéronef. Par exemple, ces capteurs 12 sont disposés de manière fixe et/ou amovible dans le cockpit de l'aéronef piloté par l'opérateur.

Chaque capteur 12 permet de fournir un type de données qui est déterminé en fonction de la nature de ce capteur 12.

Ainsi, par exemple, chaque capteur 12 permet de fournir un type de données choisi dans le groupe comprenant au moins :
- Rythme cardiaque, par exemple à des fréquences différentes (60 Hz ou 120 Hz par exemple) ;
- Saturation en oxygène ;
- Électroencéphalogramme ;
- Fréquence respiratoire ;
- Température corporelle ;
- Images ;
- Données acoustiques ;
- Activité oculaire (pupillométrie, ouverture des yeux, direction du regard) ;
- Position et accélération de la tête ;
- Niveaux de glucose sanguin ;
- Conductance cutanée (ou GSR de l'anglais « Galvanic Skin Response ») ;
- Activité musculaire (électromyogramme, EMG) ;
- Photopléthysmographie (PPG) ;
- Spectroscopie proche infra-rouge fonctionnelle (ou fNIRS de l'anglais « Functional near-infrared spectroscopy »).

Avantageusement, chaque type de données correspond à une donnée physiologique mesurable en relation avec le corps humain ou à l'environnement dans lequel il se trouve.

En référence à la figure 1, le système de détermination 10 comprend un module d'entrée 21, un module de traitement 22 et un module de sortie 23.

Le module d'entrée 21 permet d'acquérir l'ensemble des données générées par les capteurs 12. Pour ce faire, le module d'entrée 21 est connecté à chacun de ces capteurs par tout moyen techniquement possible. Ce moyen peut présenter des câbles de connexion directs ou alors indirects permettant par exemple de transmettre les données physiologiques acquises par le capteur correspondant via un réseau informatique filaire ou sans fil.

Le module d'entrée 21 est connecté en outre à une base de données 25 permettant de stocker une matrice de correspondance et une matrice d'incidence dont les significations seront expliquées plus en détail par la suite.

Le module de traitement 22 permet de traiter l'ensemble des données acquises par le module d'entrée 21 afin de déterminer des états physiologiques de l'opérateur.

En outre, le module de traitement 22 permet de déterminer les matrices de correspondance et d'incidence comme cela sera expliqué plus en détail par la suite.

Enfin, le module de sortie 23 permet de fournir les résultats de traitement du module de traitement 22 à tout système intéressé.

Un tel système intéressé comprend par exemple une interface d'interaction homme-machine ou tout autre système externe pour lequel les états physiologiques de l'opérateur présentent un intérêt.

Chacun des modules 21 à 23 présente avantageusement au moins partiellement un logiciel mis en œuvre par un processeur et stocké dans une mémoire.

Dans un tel cas, le système de détermination 10 comprend en outre un tel processeur ainsi qu'une telle mémoire.

Alternativement ou de manière optionnelle, chacun de ces modules 21 à 23 présente au moins partiellement un circuit logique programmable, par exemple de type FPGA (de l'anglais « Field Programmable Gate Array »).

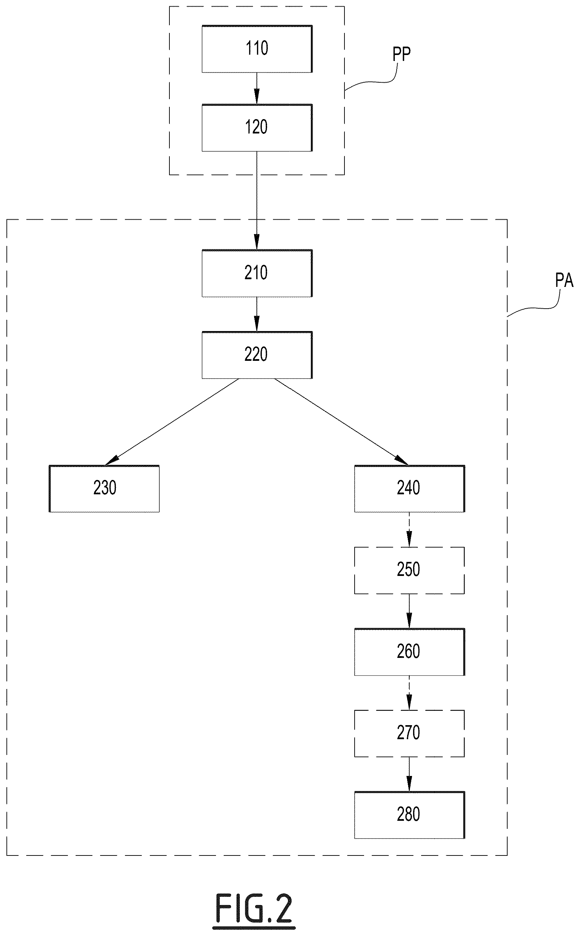

Le système de détermination 10 permet de mettre en œuvre un procédé de détermination des états physiologiques de l'opérateur qui sera désormais expliqué en référence à la figure 2 présentant un organigramme de ces étapes.

Le procédé de détermination comprend tout d'abord une phase préliminaire PP lors de laquelle les matrices de correspondance et d'incidence sont déterminées.

En particulier, lors d'une première étape préliminaire 110, le module de traitement 22 définit un modèle mathématique permettant de déterminer un état physiologique de l'opérateur et un ensemble de types de données nécessaires à la mise en œuvre de ce modèle mathématique.

Cela est fait alors en analysant chaque modèle mathématique utilisé pour déterminer l'état physiologique correspondant. Chaque modèle mathématique est par exemple connu en soi et peut être obtenu de manière théorique et/ou empirique.

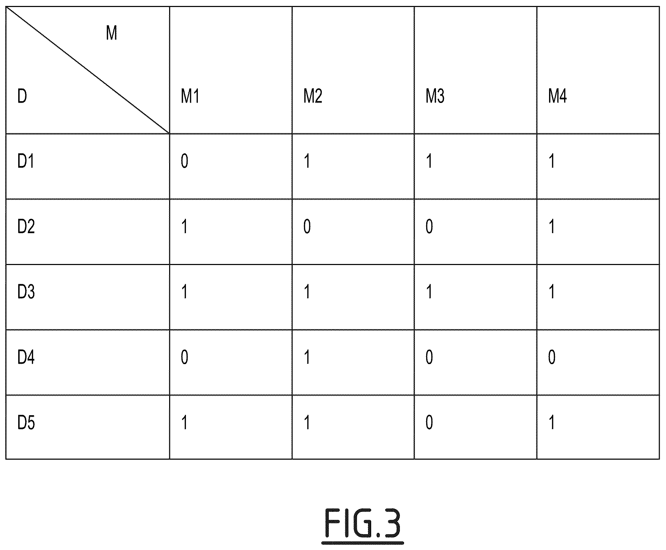

Un exemple d'une telle matrice de détermination est illustré sur la figure 3.

En particulier, dans l'exemple de cette figure 3, la matrice de correspondance comprend quatre modèles mathématiques formant alors les quatre colonnes de ces matrices permettant de déterminer des états physiologiques de l'opérateur.

Ces modèles sont numérotés de M1 à M4 et correspondent par exemple aux modèles de détermination de l'hypoxie, de la perte de conscience, du stress, et de la fatigue.

Les colonnes de cette matrice de correspondance sont formées par le type de données utilisables pour mettre en œuvre le modèle mathématique correspondant.

Dans l'exemple de la figure 3, cinq types de données numérotés de D1 à D5 sont représentés. Ces types de données correspondent par exemple respectivement au rythme cardiaque à 60 Hz, au rythme cardiaque à 120 Hz, à la saturation en oxygène, à l'électroencéphalogramme et à la fréquence respiratoire.

Selon l'exemple de la figure 3, le premier modèle M1 permet alors de détecter l'état de l'hypoxie à partir des données D2, D3 et D5 correspondant respectivement au rythme cardiaque à 120 Hz, à la saturation en oxygène et à la fréquence respiratoire.

Selon le même exemple, le modèle de détermination d'une perte de conscience, i.e. le deuxième modèle M2, permet de déterminer une telle perte de conscience en fonction des données D1, D3, D4 et D5 correspondant respectivement au rythme cardiaque à 60 Hz, à la saturation en oxygène, à l'électroencéphalogramme et à la fréquence respiratoire.

Autrement dit, lorsqu'un type de données est nécessaire pour mettre en œuvre le modèle mathématique correspondant, la matrice de correspondance indique cela dans le croisement de la ligne et la colonne correspondante, en mettant par exemple une valeur « 1 ». Autrement, une valeur zéro est alors placée dans un tel croisement.

Lors d'une deuxième étape préliminaire 120, le module de traitement 22 détermine une matrice d'incidence indiquant le niveau d'incidence de chaque état physiologique à chaque autre état physiologique.

Autrement dit, une telle matrice d'incidence permet de voir les corrélations entre les différents états, et notamment, permet de déterminer les cas lorsqu'un état physiologique induit un autre état physiologique selon un niveau d'incidence. Un tel niveau d'incidence présente par exemple une valeur comprise entre 0 et 1 et correspondant à la probabilité avec laquelle un premier état physiologique induit un deuxième état physiologique.

Alternativement, une telle valeur peut présenter le pourcentage de cas lorsque le premier état physiologique induit le deuxième état physiologique.

Lorsque le niveau d'incidence est égal à « 0 », le premier état n'induit pas le deuxième état. Autrement dit, le deuxième état est indépendant du premier état.

Lorsqu'au contraire, ce niveau d'incidence est égal à « 1 », le premier état induit toujours le deuxième état. Par exemple, il peut par exemple s'agir d'un même état ou alors des états physiologiques qui surviennent systématiquement en même temps.

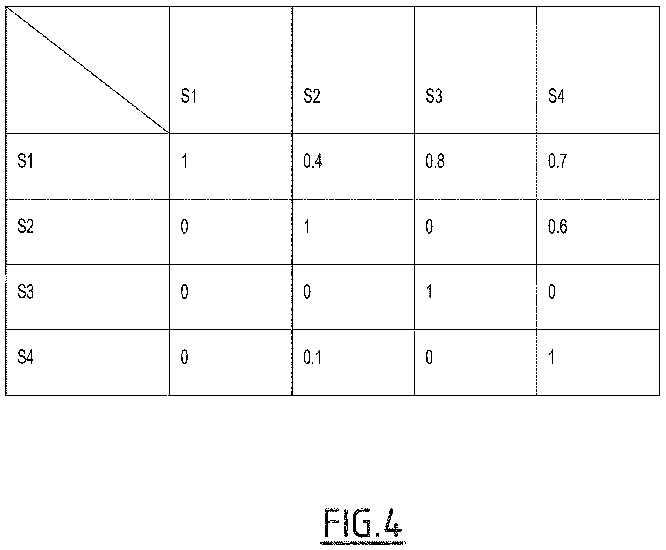

Un exemple d'une telle matrice d'incidence est donné sur la figure 4.

Dans l'exemple de cette figure, il est alors clair que l'état de l'hypoxie, correspondant à l'état S1, induit l'état de la perte de conscience (état S2) avec la probabilité 0,4, le stress (état S3) avec la probabilité 0,8 et la fatigue (état S4) avec la probabilité 0,7.

La matrice d'incidence est par exemple déterminée en analysant des données statistiques en fonction de leur nature et en fonction par exemple de la nature de la mission de l'opérateur. Une telle analyse peut par exemple comprendre la détermination de corrélations entre différents états physiologiques de l'opérateur.

Les deux étapes préliminaires 110 et 120 sont donc mises en œuvre au moins une fois avant les autres étapes du procédé de détermination.

Alternativement, ces deux étapes sont mises en œuvre à chaque mise à jour du système de détermination 10 lorsque par exemple un nouvel état et/ou un nouveau capteur sont intégrés dans un tel système.

Les étapes suivantes du procédé forment alors une phase d'analyse PA permettant de déterminer les états physiologiques de l'opérateur en utilisant les matrices telles que définies ci-dessus. Ces deux matrices sont par exemple stockées dans la base de données 25 et alors accessibles à tout moment par le module d'entrée 21.

Lors d'une étape initiale 210 de la phase d'analyse PA, le module d'entrée 21 reçoit l'ensemble des mesures fournies par les capteurs 12.

Puis, dans certains modes de réalisation, le module d'entrée 21 met en œuvre un prétraitement de ces données.

Un tel prétraitement peut comprendre un filtrage de données afin d'éliminer des bruits éventuels, ainsi que tout autre type de prétraitement analogique et/ou numérique connu en soi dans l'état de la technique.

Selon certains modes de réalisation, lors de cette étape, le module d'entrée 21 effectue en outre un prétraitement contextuel des données fournies par les capteurs pour déterminer un certain nombre de caractéristiques relatives à ces données.

Ces caractéristiques peuvent comprendre par exemple des paramètres tels que la variabilité de la fréquence cardiaque, les pics de fréquence cardiaque, les variations de la saturation en oxygène, etc.

Ce type de traitement contextuel est également connu en tant que tel et ne sera pas expliqué par la suite.

En outre, par la suite, par une donnée fournie par un capteur, on entend soit une donnée brute fournie par le capteur et convertie éventuellement par le module d'entrée 21 en une donnée numérique, soit une caractéristique générée par ce module d'entrée 21 à partir des données brutes fournies par le capteur correspondant.

En tout état de cause, la nature des données générées par le module d'entrée 21 (c'est-à-dire soit les données brutes, soit les caractéristiques) dépend de chaque modèle mathématique utilisé par le module de traitement comme cela sera expliqué plus en détail par la suite.

Lors d'une étape suivante 220, le module de traitement 22 reçoit l'ensemble de données générées et/ou reçues par le module d'entrée 21 et vérifie la disponibilité de chaque capteur 12.

Pour cela, le module de traitement 22 peut par exemple analyser la cohérence des données fournies par chaque capteur et lorsque ces données sont incohérentes, déterminer qu'il s'agit alors d'un capteur défaillant qui est alors reconsidéré comme indisponible par la suite.

Le module de traitement 22 peut déterminer en outre qu'un capteur est indisponible lorsque aucune donnée n'a été fournie par un tel capteur.

Alternativement ou en complément, le module de traitement 22 conclut qu'un capteur est indisponible en utilisant toute autre information pouvant être transmise par des systèmes externes.

Ainsi, par exemple, un système de surveillance de fonctionnement des capteurs séparés peut être utilisé pour fournir l'état de fonctionnement de chaque capteur au module de traitement 22.

Encore alternativement, l'opérateur peut lui-même indiquer un capteur défaillant de sorte qu'il est considéré ensuite par le module de traitement 22 comme un capteur indisponible.

Lorsque tous les capteurs sont disponibles, le module de traitement 22 détermine des états physiologiques à partir de chaque modèle mathématique en utilisant alors l'ensemble des données fournies par le module d'entrée 21.

Ensuite, lors d'une étape 230, le module de traitement 22 transmet ces données au module de sortie 23 qui fournit alors les états déterminés à tout système intéressé.

Lorsqu'au contraire, au moins l'un des capteurs est considéré comme indisponible, le module de traitement 22 met en œuvre l'étape 240 lors de laquelle le module de traitement 22 exclut de toute future considération chaque modèle mathématique utilisant le type de données correspondant à un tel capteur indisponible.

Pour cela, le module de traitement 22 utilise la matrice de correspondance qui indique pour chaque type de données, les modèles mathématiques utilisant ce type de données pour déterminer les états physiologiques correspondants.

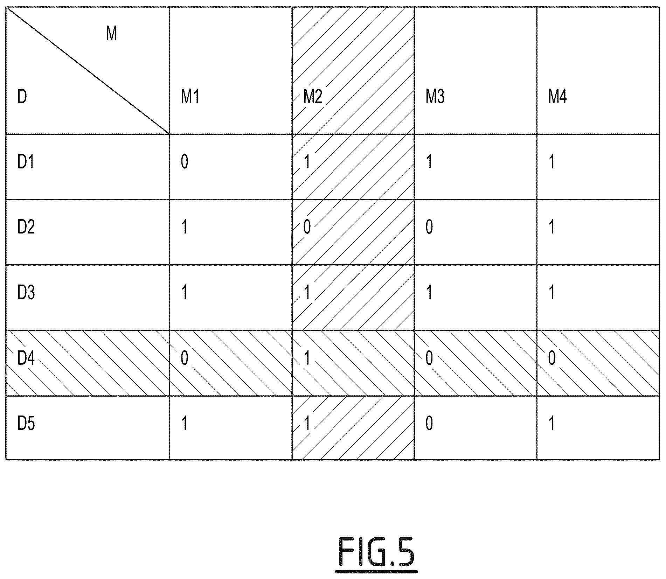

Dans l'exemple de la figure 5, le capteur fournissant le type de données D4 est considéré indisponible.

Ainsi, lors de l'étape 240, le module de traitement 22 exclut de considération le modèle mathématique M2 correspondant au seul modèle utilisant le type de données D4.

Lors d'une étape optionnelle suivante 250, le module de traitement 22 substitue le ou chaque modèle mathématique exclu de considération lors de l'étape 240 par un sous-modèle permettant d'éviter l'utilisation du type de données devant être fourni par le ou chaque capteur indisponible.

La mise en œuvre de cette étape 250 est alors conditionnelle en fonction du modèle mathématique exclu de considération lors de l'étape 240.

En particulier, lorsqu'un tel modèle exclu de considération permet de définir un sous-modèle qui n'utilise pas le type de données devant être fourni par le capteur indisponible, l'étape 250 peut être mise en œuvre en relation avec ce sous-modèle.

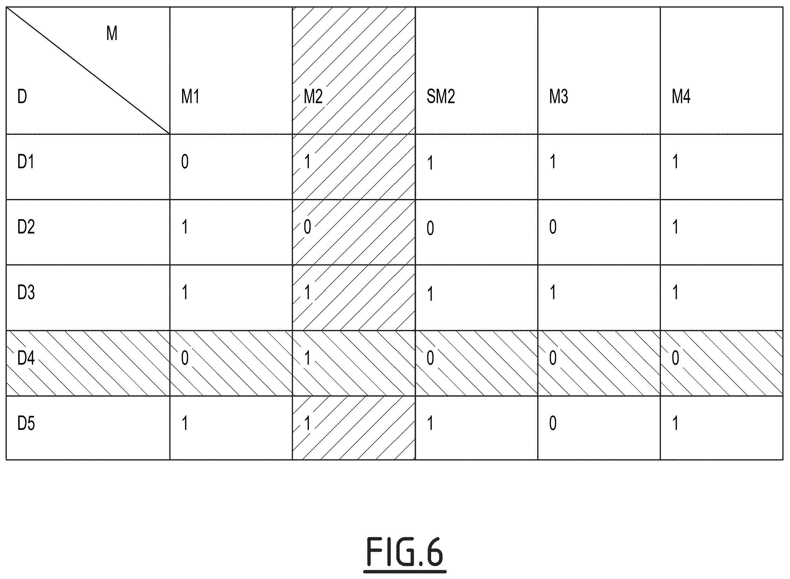

La figure 6 illustre un tel exemple dans lequel un sous-modèle SM2 a été ajouté dans la matrice de correspondance qui peut substituer le modèle mathématique M2 lorsque le type de données D4 est indisponible.

Lors de l'étape suivante 260, le module de traitement 22 détermine des états physiologiques de l'opérateur à partir des modèles mathématiques non-exclus de considération.

En particulier, lorsqu'aucun modèle mathématique exclu de considération lors de l'étape 240 n'a été substitué par un sous-modèle lors de l'étape 250, le module de traitement 22 utilise un nombre diminué des modèles pour déterminer les états physiologiques correspondants.

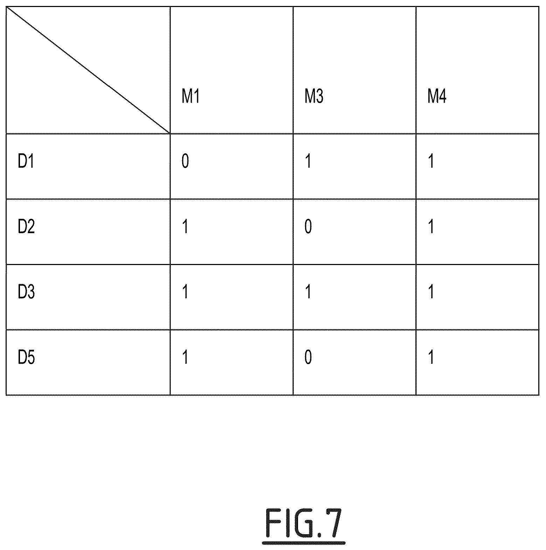

Cela est illustré dans l'exemple de la figure 7 sur laquelle seuls les modèles mathématiques M1, M3 et M4 sont utilisés à la suite de l'exclusion de considération du modèle M2.

Lorsque, suite à l'exclusion d'un modèle mathématique, le module de traitement 22 a substitué ce modèle exclu par un sous-modèle, le module de traitement 22 détermine alors lors de cette étape 260 les états physiologiques à partir de l'ensemble des modèles mathématiques disponibles et de sous-modèles déterminés lors de l'étape 250.

Eventuellement, l'état physiologique déterminé lors de l'étape 260 par un sous-modèle mathématique peut être marqué de manière correspondante. Autrement dit, un marquage cet état physiologique indiquant le fait qu'il a été déterminé à partir d'un sous-modèle et non le modèle habituel, peut être utilisé.

Lors de l'étape optionnelle 270 suivante, le module de traitement 22 détermine au moins un état physiologique correspondant à un modèle mathématique exclu de considération lors de l'étape 240 et non remplacé par un sous-modèle lors de l'étape 250.

Pour ce faire, le module de traitement 22 utilise le ou chaque état physiologique induisant l'état physiologique correspondant au modèle mathématique exclu, conformément à la matrice d'incidence expliquée précédemment.

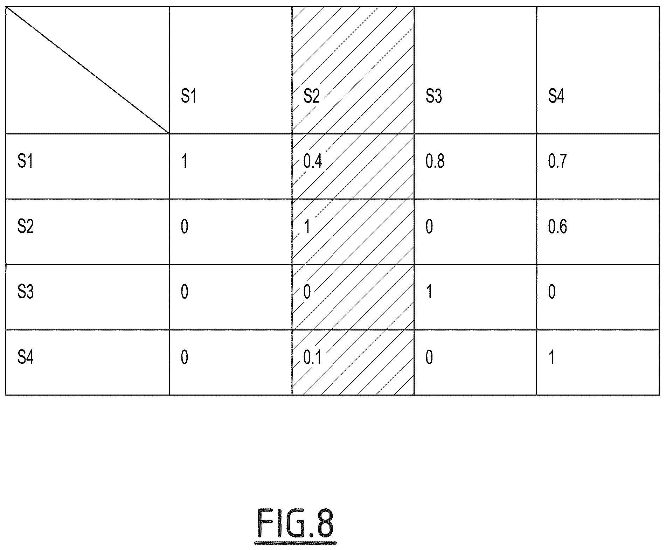

Dans l'exemple de la figure 8, l'état physiologique S2 ne peut pas être déterminé par le modèle mathématique correspondant, c'est-à-dire par le modèle mathématique M2 qui a été alors exclu suite à la perte du type de données D4.

Toutefois, dans un tel cas, le module de traitement 22 peut utiliser l'état physiologique S1 et l'état physiologique S4 qui ont respectivement le niveau d'incidence sur l'état physiologique S2 égal à 0,4 et 0,1.

Pour déterminer l'état physiologique à partir des états physiologiques induisant un tel état selon la matrice d'incidence, le module de traitement 22 utilise par exemple un modèle d'agrégation conçu à cet effet.

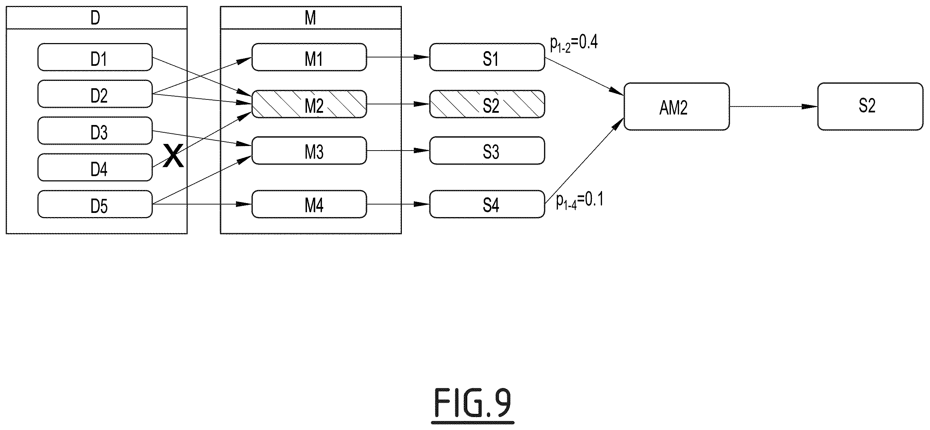

Cela peut alors être illustré schématiquement sur la figure 9, sur laquelle suite à la perte du capteur fournissant le type de données D4, le modèle mathématique M2 ne peut pas être utilisé pour déterminer l'état physiologique S2. Dans un tel cas, les états physiologiques S4 et S1 sont utilisés par un modèle d'agrégation AM2 pour obtenir un état physiologique S2.

Avantageusement, lors de cette même étape 270, le module de traitement 22 détermine en outre un niveau de confiance dudit d'état physiologique déterminé en utilisant des autres états physiologiques.

Ce niveau de confiance est par exemple déterminé en fonction du niveau d'incidence du ou de chaque état physiologique utilisé par le modèle d'agrégation correspondant.

Dans l'exemple de la figure 9, le niveau de confiance déterminé pour l'état physiologique S2 dépend alors des niveaux d'incidence p₁₋₂ et p₁₋₄ déterminés conformément à la matrice d'incidence.

Finalement, lors d'une étape suivante 280, le module de sortie 23 transmet l'ensemble des états physiologiques déterminés à tout système intéressé.

En outre, lorsqu'au moins un état physiologique a été déterminé en utilisant au moins un autre état physiologique par un modèle d'agrégation, le module de sortie 23 fournit en outre le niveau de confiance associé à cet état physiologique. De manière analogue, lorsqu'au moins un état physiologique a été déterminé en utilisant un sous-modèle à la place du modèle correspondant, le module de sortie 23 fournit en outre le marquage correspondant.

Ensuite, les étapes de la phase d'analyse PA peuvent être de nouveau mises en œuvre en utilisant d'autres données acquises par les capteurs 12.

La présente invention présente alors un certain nombre d'avantages.

Tout d'abord, il est clair que l'invention permet de mettre en évidence des liens entre les données fournies par les différents capteurs et les états physiologiques qui peuvent être déterminés à partir de ces données. Il est clair par ailleurs qu'il s'agit des états physiologiques différents.

Ainsi, en cas de l'indisponibilité d'au moins l'un des capteurs, l'invention permet d'exclure ou alors de remplacer facilement le modèle mathématique utilisant le type de données issu de ce capteur pour déterminer l'état physiologique correspondant.

Cela présente alors une grande flexibilité de l'invention par rapport aux solutions connues dans l'état de l'art.

En outre, l'invention permet d'établir des liens entre les différents états et en cas de l'incapacité de calcul de l'un des états par le modèle mathématique correspondant d'utiliser les autres états pour alors estimer ce dernier état.

Dans un tel cas, un niveau de confiance peut être fourni afin d'avertir tout autre système intéressé que l'état physiologique correspondant a été obtenu par des moyens indirects.

## Revendications

1. Procédé de détermination d'états physiologiques d'un operateur ;
le procédé comprenant une première étape préliminaire (110) de détermination d'une matrice de correspondance entre un modèle mathématique permettant de déterminer un état physiologique de l'opérateur et un ensemble de types de données nécessaires à la mise en œuvre du modèle mathématique ;
le procédé comprenant en outre les étapes suivantes :
- vérification (220) de la disponibilité de chaque capteur (12) permettant de fournir un type de données ;
- lorsqu'un capteur (12) est indisponible, exclusion (240) de considération de chaque modèle mathématique utilisant le type de données correspondant à ce capteur (12), conformément à la matrice de correspondance ;
- détermination (260) des états physiologiques de l'opérateur à partir des modèles mathématiques non-exclus de considération.

2. Procédé selon la revendication 1, comprenant en outre une étape (250) de substitution d'au moins un modèle mathématique exclu de considération par un sous-modèle mathématique permettant de déterminer le même état physiologique à partir d'un ensemble de types de données sans le type de données du capteur (12) indisponible correspondant.

3. Procédé selon l'une quelconque des revendications précédentes, comprenant en outre une deuxième étape préliminaire (120) de détermination d'une matrice d'incidence indiquant le niveau d'incidence de chaque état physiologique sur chaque autre état physiologique.

4. Procédé selon la revendication 3, dans lequel chaque niveau d'incidence correspond à la probabilité de passage d'un état physiologique à un autre état physiologique.

5. Procédé selon la revendication 3 ou 4, comprenant en outre une étape (270) de détermination d'au moins un état physiologique correspondant à un modèle mathématique exclu de considération, par un modèle d'agrégation utilisant le ou chaque état physiologique induisant cet état physiologique correspondant au modèle mathématique exclu de considération, conformément à la matrice d'incidence.

6. Procédé selon la revendication 5, dans lequel ledit état physiologique correspondant au modèle mathématique exclu de considération est déterminé avec un niveau de confiance déterminé en fonction du niveau d'incidence du ou de chaque état physiologique utilisé par le modèle d'agrégation.

7. Procédé selon l'une quelconque de revendications précédentes, dans lequel chaque état physiologique est choisi dans le groupe comprenant au moins :
- l'hypoxie ;
- la perte de conscience ;
- le stress ;
- la fatigue ;
- la désorientation spatiale ;
- le voile noir ;
- la déshydratation ;
- la charge mentale ;
- la divagation attentionnelle ;
- l'hyperventilation ;
- l'hypoglycémie ;
- la tunnelisation visuelle ou attentionnelle.

8. Procédé selon l'une quelconque de revendications précédentes, dans lequel chaque type de données est choisi dans le groupe comprenant au moins :
- Rythme cardiaque, notamment à des fréquences différentes ;
- Saturation en oxygène ;
- Électroencéphalogramme ;
- Fréquence respiratoire ;
- Température corporelle ;
- Images ;
- Données acoustiques ;
- Activité oculaire ;
- Position et accélération de la tête ;
- Niveaux de glucose sanguin ;
- Conductance cutanée ;
- Activité musculaire ;
- Photopléthysmographie ;
- Spectroscopie proche infra-rouge fonctionnelle.

9. Programme d'ordinateur comportant des instructions logicielles qui, lorsqu'elles sont exécutées par un ordinateur, mettent en œuvre un procédé selon l'une quelconque des revendications précédentes.

10. Système de détermination (10) d'états physiologiques d'un operateur comprenant des moyens techniques (21, 22, 23) configurés pour mettre en œuvre un procédé selon l'une quelconque des revendications 1 à 8.
